(19) Europäisches Patentamt
European Patent Office
Office européen des brevets

(11) EP 2 307 380 B1

(12) EUROPEAN PATENT SPECIFICATION

(45) Date of publication and mention of the grant of the patent:
**24.10.2012 Bulletin 2012/43**

(21) Application number: **09772373.8**

(22) Date of filing: **26.06.2009**

(51) Int Cl.:
***C07D 213/82*** (2006.01) ***A61K 31/455*** (2006.01)
***A61P 9/06*** (2006.01) ***A61P 9/10*** (2006.01)
***A61P 9/14*** (2006.01)

(86) International application number:
**PCT/EP2009/058017**

(87) International publication number:
**WO 2010/000673 (07.01.2010 Gazette 2010/01)**

# (54) N'-NITROXYALKYLNICOTINAMIDES FOR THE TREATMENT OF CARDIOVASCULAR DISEASES

N'-NITROXYALKYLNICOTINAMIDE ZUR BEHANDLUNG VON HERZKREISLAUFKRANKHEITEN

N'-NITROXYALKYLNICOTINAMIDES POUR LE TRAITEMENT DE MALADIES CARDIOVASCULAIRES

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **01.07.2008 PL 38556108**

(43) Date of publication of application:
**13.04.2011 Bulletin 2011/15**

(73) Proprietors:
- **Politechnika Lódzka**
  **90-924 Lódz (PL)**
- **Uniwersytet Jagiellonski**
  **31007 Krakow (PL)**

(72) Inventors:
- **GEBICKI, Jerzy**
  **PL-94-217 Lódz (PL)**
- **MARCINEK, Andrzej**
  **PL-91-496 Lódz (PL)**
- **CHLOPICKI, Stefan**
  **PL-30-047 Kraków (PL)**
- **ADAMUS, Jan**
  **PL-94-047 Lódz (PL)**
- **URBANIAK, Malgorzata**
  **PL-93-328 Lódz (PL)**

(74) Representative: **Sitkowska, Jadwiga**
**Kancelaria Patentowa**
**Al. Komisji Edukacji Narodowej**
**No. 83/106**
**PL-02-777 Warszawa (PL)**

(56) References cited:
**WO-A-2005/067927 CA-A1- 1 088 944**

- **HOOGEWERF A J ET AL: "Glycosaminoglycan Binding Assays" METHODS IN MOLECULAR BIOLOGY, HUMANA PRESS INC, NJ, US, vol. 138, 1 January 2000 (2000-01-01), pages 173-177, XP009122937 ISSN: 1064-3745**
- **SCATENA ROBERTO ET AL: "Nitric oxide donor drugs: an update on pathophysiology and therapeutic potential." EXPERT OPINION ON INVESTIGATIONAL DRUGS JUL 2005, vol. 14, no. 7, July 2005 (2005-07), pages 835-846, XP002547084 ISSN: 1744-7658**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 307 380 B1

## Description

## FIELD OF THE INVENTION

The invention relates to novel N'-nitroxyalkylnicotinamide derivatives and pharmaceutical compositions containing the same, that may be useful in the treatment of diseases of the cardiovascular system.

## BACKGROUND OF THE INVENTION

The patent description DE 2714713 discloses nicotinamide derivatives substituted with a nitroxyalkyl group at the amide nitrogen, as medicaments for the treatment of diseases of the cardiovascular system, including arterial hypertension, peripheral, central, cerebrovascular and renal blood flow disorders, arrhythmia, ischaemic heart disease, having also anticoagulative action. Among these compounds, N'-(2-nitroxyethyl)nicotinamide, known under the generic name of nicorandil, found its use in medicine as a medicament for the treatment of, among others, ischaemic heart disease. This compound has a dual vasodilatating action, combining the nitrate action - as a donor of nitric oxide $NO^-$ - with an ability to increase the membrane conduction of $K^+$ *via* activation of potassium channels and to increase the concentration of cGMP. Nicorandil releases NO' while being metabolised in the body into N'-(2-hydroxyethyl)nicotinamide which is then further metabolised into nicotinamide and nicotinic acid. It is believed that these metabolites, nicotinamide and nicotinic acid, are associated with the side effects comprising ulcerations appearing at various places of the body of the patients receiving this medicament (especially aphthae and oral cavity ulcerations), what makes it necessary to discontinue the treatment with this medicament (Buxton, G.V.; Greenstock, C.L.; Helman, W.P. J. Phys. Chem. Ref. Data, 1988, 17, 513).

Use of certain quaternary pyridinium salts, in particular 1-methylnicotinamide (MNA), as anti-inflammatory and vasoprotective agents useful *inter alia* in the treatment of skin diseases, thrombosis, atherosclerosis and hyperlipidaemia is known from WO/2000/040559 and WO/2005/067927. It is believed that MNA exerts its vasoprotective action by binding to vascular endothelium, namely to the glycosaminoglycan receptors present at the endothelium, through which MNA biological function of modulating the secretory activity of the endothelium is effected (Gebicki, J.; Sysa-Jedrzejowska A.; Adamus, J.; Woźniacka, A.; Rybak, M.; Zielonka, J. Pol. J. Pharmacol., 2003, 55, 109-112). MNA is characterised by a lack of toxicity and a good tolerance in organism, after both external and systemic administration.

Thus, there is a need for compounds that, while exhibiting an advantageous pharmacological profile as desired in the case of medicaments for the treatment of diseases of the cardiovascular system, would also have a smaller tendency to induce side effects caused by metabolites.

Unexpectedly, it was found that such an advantageous and desired pharmacological profile could be displayed by the compounds according to the invention, novel N'-nitroxyalkylnicotinamide derivatives, that are characterised by a double pharmacological activity - a nitric oxide donor activity characteristic for nitrates as well as vasoprotective activity, characteristic for MNA. They are characterised also by a lack of ability to undergo biotransformation towards unwanted metabolites of the known N'-(2-nitroxyethyl)nicotinamides, namely nicotinamide and nicotinic acid, and consequently, by a reduced tendency to cause side effects.

## DETAILED DESCRIPTION OF THE INVENTION

Thus, the invention provides novel compounds, N'-nitroxyalkylnicotinamide derivatives, represented by the general formula I:

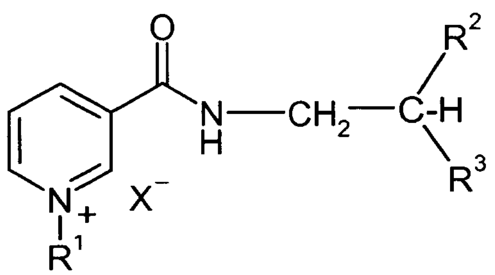

I

wherein:

$R^1$ is $C_1$-$C_4$alkyl;

$R^2$ is hydrogen, $C_1$-$C_4$ alkyl, $CH_2OH$ or $CH_2ONO_2$;

$R^3$ is $ONO_2$, $CH_2ONO_2$ or OH,

provided that when $R^3$ is OH, $R^2$ is $CH_2ONO_2$; and

$X^-$ is an organic or inorganic anion.

Novel compounds according to the invention show biological activity and can be useful as active substances of medicaments.

The invention provides also the compounds of the above-defined formula I for use as medicaments.

The invention provides also a pharmaceutical composition comprising a compound of the formula I as defined above as an active substance, in combination with pharmaceutically acceptable conventional carriers and/or auxiliary substances.

The invention provides also the use of a compound of the formula I as defined above for the manufacture of a medicament for the treatment and prevention of diseases of the cardiovascular system.

Also disclosed is a method of treatment and/or prevention of diseases of the cardiovascular system, comprising the administration of an effective amount of a compound of the formula I as defined above to a subject in need of such treatment and/or prevention.

Preferred compounds of the above formula I are the compounds wherein $R^1$ is methyl.

Also preferred are the compounds of the above formula I wherein $R^2$ is hydrogen and $R^3$ is $ONO_2$.

Another group of the preferred compounds of the above formula I are the compounds wherein X is Cl (chloride) anion.

One example of the particular compounds according to the invention are 1-methyl-N'-(2-nitroxymethyl)nicotinamide salts, especially 1-methyl-N'-(2-nitroxymethyl)nicotinamide chloride.

The term $C_1$-$C_4$ alkyl in the above formula I comprises straight-chained and branched alkyl groups, in particular methyl, ethyl, n-propyl, isopropyl, n-butyl, *sec*-butyl and *tert*-butyl, especially methyl.

In the compounds according to the invention, the anion $X^-$ may be any salt-forming organic or inorganic anion. Preferably, the salt-forming anion $X^-$ is a pharmaceutically acceptable organic or inorganic anion, *i.e.*, an anion having no toxic or otherwise harmful effect on the body, in particular being acceptable for oral consumption and administration by injections. However, the invention covers also the compounds of the formula I wherein the anion $X^-$ is not a pharmaceutically acceptable one; such compounds may be useful in the processes of preparation and/or purification of the compounds wherein $X^-$ is a pharmaceutically acceptable anion.

The non-limiting examples of pharmaceutically acceptable inorganic anions $X^-$ are halide anions, including chloride, bromide and iodide anion, and carbonate anion. The non-limiting examples of pharmaceutically acceptable organic anions $X^-$ are the anions of aliphatic or aromatic mono-, di- and tricarboxylic acids, such as acetate, benzoate, salicylate, hydroxyacetate, lactate, malonate, citrate, or the like

The pharmaceutically advantageous anion $X^-$ is the chloride anion.

It should be understood, as it will be appreciated by a person skilled in the art, that when the compound of the formula I is a component of a pharmaceutical composition and/or is used as a medicament in the treatment of the diseases described herein, $X^-$ in the formula I will be a pharmaceutically acceptable anion.

The compounds according to the invention may be prepared from the corresponding compounds not substituted with an alkyl group at the pyridine ring nitrogen atom, that is at the position 1 of the ring, *i.e.*, the compounds of the formula II

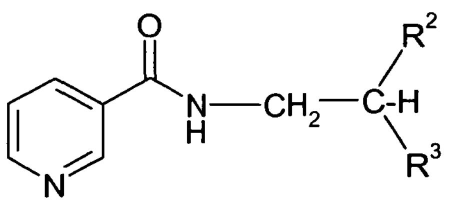

II

wherein $R^2$ and $R^3$ are as defined for the formula I.

In the case where $X^-$ is a halide anion, the compounds of the formula I can be obtained by direct alkylation, in a manner well known to the persons skilled in the art *per se,* of the compound of the formula II as defined above with an alkyl halide of the formula Hal-$R^1$ wherein Hal is a halide anion, and $R^1$ is as defined above for the formula I.

The compounds wherein the halide anion $X^-$ is a chloride anion can be also obtained by treating the compound of the above formula II with an alkyl iodide, to yield the compound of the formula I, wherein $X^-$ is an iodide anion, followed

by exchange of iodide ion for chloride anion by the treatment with silver chloride.

The compounds wherein the halide anion $X^-$ is the chloride anion can be also prepared by treating the compound of the formula II with an alkyl chloride $R^1Cl$, as described *for example,* in the Austrian patent publication No. AT 131118, the British patent description No. GB348345, the U.S. patent description No. US 3614408, and the U.S. patent description No. US 4115390.

The compounds of the formula I wherein the anion $X^-$ is other than a halide anion can be obtained from the compound of the formula I wherein $X^-$ is a halide anion by substitution of the halide anion with another anion, for example, by the treatment with a silver salt of that another anion. For example, the compounds of the formula I wherein $X^-$ is lactate, benzoate or acetate can be obtained by treating the compound of the formula I wherein $X^-$ is a halide anion, preferably chloride anion, with silver acetate, benzoate or lactate, respectively.

The starting compounds of the formula II can be prepared as described in the German patent description No. DE 2714713, by reacting nicotinic acid or its functional derivative at the carboxyl group, such as acid halide, acid anhydride, active ester or active amide, with an amine compound of the formula III

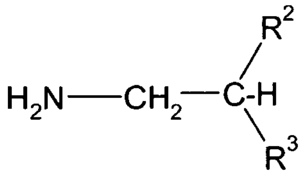

III

wherein $R^2$ and $R^3$ are as defined for the formula I, or an active form of a compound of the formula III, i.e. the compound of the formula III activated at the amino group the use of phosphorus trichloride, methyl or ethyl chlorophosphite, or the like, at a temperature ranging from -10°C to 50°C, in water or an inert organic solvent, such as, for example, benzene, toluene, tetrahydrofuran, dioxane, dimethylformamide, chloroform, methylene chloride, acetonitrile, acetone, ethyl acetate, or the like. The reaction may be carried out in water or an inert organic solvent in the presence of an alkaline inorganic substance, such as, for example, an alkali metal or alkaline earth metal hydroxide, carbonate or acetate, such as sodium acetate, sodium carbonate, sodium hydroxide, potassium acetate, potassium carbonate, potassium hydroxide; or in the presence of an amine compound, such as, for example, pyridine, triethylamine, dimethylaniline, picoline, or the like. The reaction may be carried out also in an organic alkaline solvent, such as, for example, pyridine, triethylamine, dimethylaniline, picoline, or the like.

The process of the preparation of the compound of the formula II by the reaction of nicotinic acid or its functional derivative at the carboxyl group with the compound of the formula III may also be carried out in the presence of an activator: an imide compound, such as for example, N,N'-dicyclohexylcarbodiimide and the like; an imine compound, such as for example, diphenylketene-N-cyclohexylimine and the like; or a phosphate or phosphate, such as, for example, triethyl phosphate, and the like.

Alternatively, certain starting compounds of the formula II may be prepared in a process also described in the German patent description No. DE 2714713, by the reaction of an amide compound of the formula IV

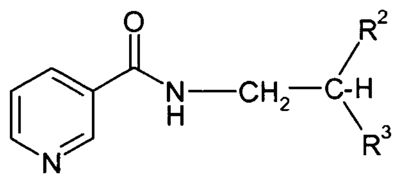

IV

wherein $R^2$ is $CH_2OH$ and/or $R^3$ is OH or $CH_2OH$, with a nitrating agent, such as fuming nitric acid, nitrating acid or sodium nitrate.

The novel compounds according to the invention exhibit a biological activity and may be useful as active substances of the medicaments.

In particular, said novel compounds are capable to release nitric oxide. Due to this they have a therapeutic potential to act as agents increasing the bioavailability and the level of nitric oxide, useful for a broad range of therapeutic indications where the nitric oxide donors are useful. They can exert a vasodilative effect on peripheral and coronary vessels, increase the coronary blood flow, and protect the myocardium. They can also exert an antithrombotic effect. Among the therapeutic indications for the compounds according to the invention that can be mentioned are hypertension and ischaemic heart disease. Besides, the inventors believe that the compounds according to the invention are also capable to bind to glycosaminoglycan receptors, due to which they show the potential for binding with vascular endothelium and modulating the endothelium functions. This may result in exerting many endothelial effects that may be beneficial from the pharmacological point of view. Such effects may comprise the release of endogeneous NO and/or prostacycline, resulting in the improvement of endothelial dysfunction and thus treatment or prevention of endothelial dysfunction derived diseases. In particular, the compounds may exhibit the hypotensive, vasodilating, anti-arrhythmic, thrombolytic, antithrombotic and anti-atherosclerotic action. Furthermore, the inventors believe that the compounds will be metabolised to well-tolerated N-methylnicotinamide, without formation of nicotinamide and nicotinic acid as intermediate metabolites, and in consequence it will be possible to avoid adverse side effects that limit the utility of prior art compounds. Also, N-methylnicotinamide formed as a metabolite will be able to exert its known actitvity as a vasoprotective agent, as described in the prior art.

Thus, the compounds according to the invention may be useful in the treatment and prevention of diseases of the cardiovascular system.

The treatment and/or prevention of diseases of the cardiovascular system will comprise the administration of an effective amount of a compound of the formula I as defined above to the subject in need of such a treatment and/or prevention.

In particular, the diseases of cardiovascular system comprise, without such limitation, arterial hypertension, coronary heart disease, angina, arrhythmia, acute and chronic ischaemic heart disease, vascular atherosclerosis, thromboses of various origin, peripheral, central, cerebral and renal blood flow disorders, including blood flow disorders and thromboses as a result of complications of diabetes mellitus.

The compounds according to the invention may be also useful in the prevention of diseases of the cardiovascular system in subjects at high risk for such diseases. The risk factors comprise the lifestyle factors, such as smoking, high level of psychical stress, sedentary lifestyle, obesity; physiological factors such as hypercholesterolemia, arterial hypertension, hyperhomocysteinemia, metabolic syndrome, insulin resistance, diabetes mellitus, menopause, age-dependent decrease of the prostacyclin synthesis, obesity, infections, inflammatory states, including parodontitis, rheumatoidal arthritis, graft atherosclerosis after transplantation of organs.

Vascular atherosclerosis comprises arterio-atherosclerosis in the subjects suffering from stable coronary heart disease, atherosclerosis in the subjects suffering from cerebral ischaemic episodes, and atherosclerosis of extremities, including Buerger's disease.

Thromboses of the origin other than arterio-atherosclerosis comprise, in particular, thrombosis related to implantation of metallic prostheses and vascular prostheses (stents); coronary artery bypass graft (CABG); haemodialysis; and venous thromboembolic disease.

The compounds according to the invention defined above may be useful in the treatment and prevention of atherosclerosis related cardiovascular episodes, in particular acute coronary syndrome (including unstable ischaemic heart disease, myocardial infarction), in the conditions requiring coronary angioplasty (percutaneous coronary intervention, PCI) or coronary artery bypass graft (CABG), and in ischaemic brain stroke. They may be also used prior to surgical procedures involving extracorporeal circulation or prior to procedures of revascularisation of peripheral circulation.

In the treatment and prevention of the diseases discussed above, the compounds according to the invention will be used in the form of pharmaceutical compositions containing at least one compound of the formula I in combination with pharmaceutically acceptable carriers and/or auxiliary substances.

The compositions may be in the form destined for oral administration. Such forms may be typical solid and liquid forms for oral administration, as known in the art, such as tablets; hard and soft capsules filled with powder or granules; granules; powders; solutions; suspensions, and the like. They will contain carriers and excipients/auxiliary substances typically used in the art for the formulation of oral dosage forms, such as fillers, tabletting aids, flavouring agents, and the like. Any of these excipients must be "acceptable" in the sense of compatibility with the other components of the formulation, in particular with the active substance, and cannot be deleterious for the patient. Non-limiting examples of materials that may be used as pharmaceutically acceptable carriers and fillers are: sugars, such as lactose, mannitol, glucose and sacharose; starches, such as maize starch and potato starch; cellulose and derivatives thereof, such as sodium carboxymethylcellulose, ethylcellulose, hydroxypropylcellulose, cellulose acetate, microcrystalline cellulose; powdered tragacanth; polyvinylpyrrolidone; calcium phosphate. The oral formulations may also contain lubricants and glidants, such as, for example, stearates, like magnesium stearate, talc or silica; disintegrants, such as, for example, sodium starch glycolate; or wetting agents, such as, for example, sodium lauryl sulphate. Tablets may be coated by typical methods with conventional coatings (dragées) or with delayed-release coatings.

Liquid forms for oral administration may be solutions, syrups or suspensions, optionally with the addition of suspending agents and pH regulating agents. A suitable carrier may be water. They may also contain typical preservatives conventionally used to prevent the microbial growth, such as, for example, parabens, ascorbic acid, thimerosal, sorbic acid, methyl or propyl p-hydroxybenzoates, and the like.

The compounds may be also administered parenterally in the form of intravenous or subcutaneous injections. The suitable carriers may be pyrogen-free water, isotonic saline, phosphate buffers, Ringer's solution, oil carriers and other non-toxic substances used in the dosage forms technology. They may contain tonicity regulators, such as sodium

chloride, sugars or polyalcohols, such as mannitol or sorbitol, as well as stabilisers and preservatives.

The compounds may be also administered by inhalation. In such a case, they may be administered in the form of micronised powder or sprayed aerosol. They may be also administered intranasally in the form of sprayed aerosol. They may be also administered rectally, in the form of creams, ointments, suppositories.

In any case, pharmaceutical compositions will contain auxiliary substances, carriers and vehicles suitable for a given pharmaceutical form.

The compositions, wherein the compounds of the formula I may be administered according to the invention, are by no means limited to the specific forms as mentioned above.

The description of typical drug dosage forms, their manufacturing techniques as well as excipients used can be found, for example, in the standard textbook Remington's: The Science and Practice of Pharmacy, 21-st edition, 2005.

Dosage of the compounds of the formula I will depend on the kind of condition or disease in question, type of treatment (therapeutic or preventive), condition and age of the subject being treated, and will be eventually adjusted individually by the attending physician. Generally, the administered amount of a compound will be in the range from 0.1 to 10000 mg, for administering in a single dose or in divided doses, like for example, from 0.5 mg to 1,125 mg, 1 mg to 1100 mg, 1.25 mg to 1075 mg, 1.5 mg to 1050 mg, 2.0 mg to 1025 mg, 2.5 mg to 1000 mg, 3.0 mg to 975 mg, 3.5 mg to 950 mg, 4.0 mg to 925 mg, 4.5 mg to 900 mg, 5 mg to 875 mg, 10 mg to 850 mg, 20 mg to 825 mg, 30 mg to 800 mg, 40 mg to 775 mg, 50 mg to 750 mg, 100 mg to 725 mg, 200 mg to 700 mg, 300 mg to 675 mg, 400 mg to 650 mg, 500 mg, or 525 mg to 625 mg. In still another embodiment, the dose of a compound contained in the administered composition will be between 0.1 mg and 25 mg. In certain embodiments, the dose of a compound contained in the administered composition will be less than 100 mg, or less than 80 mg, or less than 60 mg, or less than 50 mg, or less than 30 mg, or less than 20 mg, or less than 10 mg, or less than 5 mg, or less than 2 mg, or less than 0.5 mg.

## BRIEF DESCRIPTIONS OF THE DRAWINGS

Fig. 1 is a graph showing the vasodilatating effect of the compound of the Example 1 ($MNIC^+Cl^-$) in isolated rat aorta as a % of phenylephrine induced contraction versus concentration.

Fig. 2 is a graph showing the effect of ODQ on the vasodilatating activity of the compound of the Example 1 ($MNIC^+Cl^-$).

Fig. 3 is a graph showing the effect of GLB on the vasodilatating activity of the compound of the Example 1 ($MNIC^+Cl^-$).

Fig. 4 shows the concentration of 1-methylnicotinamide (MNA) in rats plasma after administration of MNA or the compound of the Example 1 ($MNIC^+Cl^-$).

The following examples illustrate the invention without limiting its scope.

## EXAMPLES

### Example 1

### 1-Methyl-N'-(2-nitroxyethyl)nicotinamide iodide

#### 1.1. 2-Nitroxyethylamine nitrate

A solution of monoethanolamine (7.5 ml) in $CH_2Cl_2$ (30 ml) was added dropwise to a mixture of $HNO_3$ (15.4 ml) and $CH_2Cl_2$ (150 ml), (kept at 0-5°C by cooling in an ice bath) over about one hour and the reaction mixture was stirred for further 15 minutes at the same temperature. Then an excess of acetic anhydride (20 ml) was added dropwise and the reaction mixture was stirred for further 15 minutes at room temperature. The precipitated solid was filtered on a Schott funnel and washed with $CH_2Cl_2$. It was crystallised from EtOH (60 ml) to yield 11.84 g of the pure product (white needles), having the melting point of 102-103°C.

#### 1.2. N'-(2-Nitroxyethyl)nicotinamide

15.241 g (85.6 mmol) of nicotinoyl chloride hydrochloride were added in small portions to a solution of 9.025 g (57.9 mmol) of 2-nitroxyethylamine nitrate (obtained as described above in step 1.1) in pyridine (100 ml) at about 5°C, while overpressure was maintained by introducing argon. After stirring for 30 minutes at room temperature, the reaction

mixture was evaporated to dryness by use of a vacuum pump, while pyridine was frozen in liquid nitrogen. The residue was dissolved in 250 ml of chloroform and shaken in a separatory funnel with a saturated solution of sodium hydrogen carbonate. The organic layer was separated and dried over anhydrous magnesium sulphate, then rotary evaporated to give an oily residue that crystallised after a short time. The compound was purified on a silica gel, using a 10:1 benzene-methanol mixture as an eluent, and the eluate was rotary evaporated. This purification was repeated twice. Then the compound was crystallised from a cold mixture of 60 ml of ether and 20 ml of ethanol. 1.102 g of the product having a melting point of 91-92°C was obtained. $^1$H NMR (250.13 MHz, DMSO) $\delta$: 3.61 (q), 4.60 - 4.67 (t), 7.49 (ddd, J = 0.9, 4.8, 8.0 Hz), 8.15 (ddd, J=1.7, 2.3, 8.0 Hz), 8.69 (dd, J = 1.7, 4.8 Hz), 8.89 (t), 8.96 (dd, 0.9, 2.3 Hz).

**1.3. 1-Methyl-N'-(2-nitroxyethyl)nicotinamide iodide**

1.102 g (5.2 mmol) of N'-(2-nitroxyethyl)nicotinamide (obtained as described above in step 1.2) was dissolved in 20 ml of MeOH and 2 ml (32 mmol) of methyl iodide were added. After being left for a week in the darkness, the reaction mixture was rotary evaporated to dryness to yield the title product. $^1$H NMR (250.13 MHz, DMSO) $\delta$: 3.70 (q), 4.39 (s), 4.68 (t), 8.24 (dd, J = 6.1, 8.1 Hz), 8.86 (d, J = 8.1 Hz), 9.09 (d, J = 6.1 Hz), 9.33 (t, J = 9.38 Hz).

**Example 2**

**1-Methyl-N'-(2-nitroxyethyl)nicotinamide chloride**

1-Methyl-N'-(2-nitroxyethyl)nicotinamide iodide (a dry residue prepared as described above in step 1.3) was dissolved in water, and silver chloride (freshly prepared from 14 mmol NaCl and 14 mmol $AgNO_3$) was added to the solution. The solution was stirred using a mechanical stirrer for about twenty-four hours at room temperature. Then it was filtered on a Schott funnel and rotary evaporated, to yield 1.343 g of the title product, having a melting point of 129-131°C.$^1$H NMR (250.13 MHz, DMSO) $\delta$: 3.70 (q), 4.35 (s), 4.70 (t), 8.21 (dd, J = 6.1, 8.1 Hz), 9.03 (d, J = 8.1 Hz), 9.12 (d, J = 6.1 Hz), 9.60 (s), 9.94 (t, J = 5.35 Hz). UV-VIS: $\lambda$ = 265 nm.

**Example 3**

**Binding to heparin**

**The absorption method**

The absorption method consisted in comparing the absorption spectrum of an aqueous solution of the tested compound with the absorption spectrum of the same solution after its incubation with Sepharose immobilised heparin as a glycosaminoglycan mimicking the glycosaminoglycan receptor. The absorption spectra were recorded using a Perkin Elmer Lambda 40 spectrophotometer. The following reagents were prepared prior to the experiment: an aqueous solution of the tested compound of Example 1, namely 1-methyl-N'-(2-nitroxyethyl)nicotinamide chloride ($MNIC^+Cl^-$), at a concentration of 100 $\mu$M, an aqueous suspension of heparin immobilised on Sepharose at 25 mg/ml, and a solution of $MNIC^+Cl^-$ in an aqueous suspension of heparin having the same concentrations in both the tested compound and heparin as mentioned above. Following the initial incubation of the solution of the tested compound with heparin, it was centrifuged for 6 minutes at 13,000 rpm. Then supernatant solutions above the precipitate of heparin were separated and the spectra of the solutions were recorded at the wavelength range of 200-600 nm.

In the presence of heparin, adsorption of the part of $MNIC^+$ cations on this macromolecule resulted in lowering of the concentration of the compound in the solution and reduction of the absorption was observed. The degree of binding of $MNIC^+$ to heparin was determined by calculating the areas under the absorption curves of the compound before and after contact with heparin, using the following relationship:

$$a = \frac{IA - IA_{Hep}}{IA} \cdot 100\%$$

wherein:

*IA* and $IA_{Hep}$ are areas under the curve of absorption spectra for the tested compound, before and after contact with heparin, respectively.

The degree of binding of $MNIC^+$ to heparin, as determined by this method, is 30%. The analogously determined degree of binding of 1-methylnicotinamide (MNA) is 50%.

**The radiation method**

The experiment consisted in studying the effect of the presence of heparin on the observed rate of scavenging the hydrated electron by the $MNIC^+$ cation. The studies were carried out by pulse radiolysis, using an Elektronika ELU-6E linear electron accelerator (at the Institute of Applied Radiation Chemistry, Technical University of Lodz, Poland) having an electron beam energy of 6-8 MeV, as an ionising-radiation source. A solution of water-soluble heparin at a concentration of 10 μM was used in the experiment. The experiment was carried out for 6 concentrations of the tested compound ($MNIC^+Cl^-$) (50, 100, 200, 400, 600 and 800 μM) with no heparin added, and for 6 concentrations of the tested compound (20, 60, 120, 200, 280 and 360 μM) in the presence of heparin. The effect of the presence of heparin on the observed rate of scavenging of the hydrated electron by the $MNIC^+$ cation was investigated by recording the oscilloscopic traces for $\lambda$ = 720 nm (the hydrated electron is characterised by a strong optical absorption band with a maximum at 720 nm). The hydrated electron was generated using 3 ns electron pulses, and the absorbed dose was ca. 6 Gy/pulse. In order to scavenge the simultaneously generated hydroxyl radicals, the addition of 1 M *tert*-butyl alcohol was used. All samples were degassed with argon.

Based on the recorded kinetic traces of decay of the hydrated electron in the presence of the $MNIC^+Cl^-$ solutions at various concentrations, the values of the pseudo-first order rate constants were determined. The value of the second-order rate constant for the investigated reaction was determined from the slope of the linear dependence of the above-determined rate constants *versus* concentration. This value was $4.17 \cdot 10^{10}$ $mol^{-1}dm3s^{-1}$. Then, based on the determined second-order reaction rate constant and the observed rate constant for decay of the hydrated electron in the presence of heparin and $MNIC^+$, the degree of binding of the tested compound to heparin was determined using the following relationship:

$$k_{obs} = {}^{2}k \cdot (1-\alpha) \cdot C_{MNIC}$$

wherein:

$k_{obs}$ - the observed rate constant for the decay of hydrated electron in the presence of heparin and $MNIC^+$ cation,
${}^{2}k$ - the second-order rate constant for the reaction of hydrated electron with the $MNIC^+$ cation,
$\alpha$ - the degree of binding of $MNIC^+$ cation to heparin,
$C_{MNIC}$ - concentration of the $MNIC^+$ cation.

The degree of binding of $MNIC^+Cl^-$ to heparin, as determined by this method, is 47%. The degree of binding of 1-methylnicotinamide was determined analogously and is 39%.

The results of these studies show that $MNIC^+Cl^-$ binds in a significant degree to heparin that mimicks the glycosaminoglycan receptors, thus demonstrating the potential of the compounds of the invention to bind with the vascular endothelium and affect the secretory function of the endothelium, and due to this exert the vasoprotective action.

**Example 4**

**Vasodilating effect of 1-methyl-N'-(2-nitroxyethyl)nicotinamide chloride ($MNIC^+Cl^-$)**

The vasodilating effect of $MNIC^+Cl^-$ in aqueous solutions on isolated rat aorta was studied in the conditions close to physiological ones. 3-5 mm aortal rings with preserved endothelial function placed in a set of 6 containers á 5 ml containing the Krebs-Henseleit buffer constantly aerated with 5% $CO_2$ in $O_2$ and thermostated at 37°C were suspended between two hooks connected to the force transducer for recording the ring contraction. Then, following the constriction induced by phenylephrine, the concentration-dependent vasodilating effect of $MNIC^+Cl^-$ was investigated by measurement of relaxation of phenylephrine contracted aortal rings.

The results are shown in Fig. 1 as a % of vasodilatation versus concentration of $MNIC^+Cl^-$. As it results from Fig. 1, $MNIC^+Cl^-$ exhibits the concentration-dependent vasodilating activity at the concentrations range from tens to hundreds μmol/l.

The experiments explaining the mechanisms responsible for vasodilation induced by $MNIC^+Cl^-$ were carried out in the same system of isolated vascular vessels. In order to determine the contribution of sGC and $K_{ATP}$ channels

in the vasodilating activity of the compound, the effects of an inhibitor of ATP-dependent potassium channels - glibenclamide (GLB) at a concentration of 10 μM, and an inhibitor of soluble guanyl cyclase (sGC) 1H-[1,2,4]-oxadiazole[4,3-a]quinoxalin-1-one (ODQ) at a concentration of 10 μM on this vasodilating activity were investigated in two set of experiments. In these experiments, $MNIC^+Cl^-$ was used at the concentrations causing about 80-90% vasodilation, as determined in the experiment described above.

In the first set of experiments the effect of pre-incubation of aortal rings with ODQ on the vasodilatating activity of $MNIC^+Cl^-$ was investigated. The results are shown on Fig. 2 as a % response in comparison with control experiment without addition of ODQ. As can be seen from Fig. 2, sGC inhibitor ODQ inhibited vasodilatating activity of $MNIC^+Cl^-$ and addition of GLB had no further influence on reversal of this effect.

In the second set of experiments the effect of pre-incubation of aortal rings with GLB on the vasodilatating activity of $MNIC^+Cl^-$ was investigated. The results are shown on Fig. 3 as a % response in comparison with control experiment without addition of GLB. As can be seen from Fig. 3, potassium channel inhibitor GLB had no influence on vasodilatating activity of $MNIC^+Cl^-$. However, inhibition of vasodilatating activity of $MNIC^+Cl^-$ was observed following further addition of ODQ.

Thus, the results of the experiments show clearly that the tested compound is very effective vasodilatator, acting through soluble guanyl cyclase (sGC) stimulation dependent mechanism.

Preliminary investigations of the ability of $MNIC^+Cl^-$ to release NO were also performed using EPR (Electron Paramagnetic Resonance) method. The results of these studies confirmed that $MNIC^+Cl^-$ releases NO, and most likely the vasodilating activity of the compound is a consequence of the stimulation of sGC by NO thus released.

**Example 5**

**Metabolism of 1-methyl-N'-(2-nitroxyethyl)nicotinamide chloride ($MNIC^+Cl^-$)**

To evaluate the metabolism of $MNIC^+Cl^-$, 2 groups of rats (n =3) were watered for 2 days with aqueous solutions of $MNIC^+Cl^-$ (first group) or MNA (second group), respectively. Both compounds were given in a drinking water at the daily dose of 100 mg per kg of body weight per day. After 2 days, MNA plasma levels were measured in both groups. The results are presented on Fig. 4. The presence of MNA in the plasma of rats administered with $MNIC^+Cl^-$ suggests that 1-methylnicotinamide (MNA) is a metabolite of MNIC. The literature data on the metabolism of nicorandil allow to assume that the amount of MNA would be even greater on prolonged administration of the compound.

## Claims

1. An N'-nitroxyalkylnicotinamide derivative represented by the general formula I:

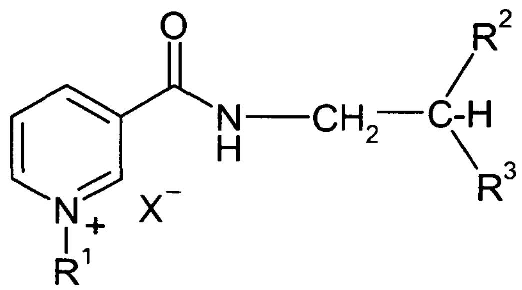

I

wherein:

$R^1$ is $C_1$-$C_4$ alkyl;
$R^2$ is hydrogen, $C_1$-$C_4$ alkyl, $CH_2OH$ or $CH_2ONO_2$;
$R^3$ is $ONO_2$, $CH_2ONO_2$ or OH;
provided that when $R^3$ is OH, $R^2$ is $CH_2ONO_2$; and
$X^-$ is an organic or inorganic anion.

2. The compound according to claim 1 wherein $R^1$ is methyl.

3. The compound according to claim 1 wherein $R^1$ is methyl, $R^2$ is hydrogen and $R^3$ is $ONO_2$.

4. The compound according to any one of claims 1 to 3 wherein X is Cl.

5. An N'-nitroxyalkylnicotinamide derivative as defined in claims 1 to 4 for use as a medicament.

6. A pharmaceutical composition, comprising an N'-nitroxyalkylnicotinamide derivative as defined in claims 1 to 4, as an active substance, in combination with a pharmaceutically acceptable carrier and/or excipients.

7. Use of an N'-nitroxyalkylnicotinamide derivative as defined in claims 1 to 4 for the manufacture of a medicament for the treatment of diseases of the cardiovascular system.

8. N'-nitroxyalkylnicotinamide derivative as defined in claims 1 to 4 for use in a method for the treatment of diseases of the cardiovascular system.

9. The use according to claim 7 or 8 wherein the disease is selected from the group comprising: arterial hypertension; coronary heart disease; acute and chronic ischaemic heart disease; arrhythmia; stable and acute angina; thrombosis; and peripheral, central, cerebral and renal blood flow disorders.

**Patentansprüche**

1. Ein N'-Nitroxyalkylnikotinamid-Derivat der allgemeinen Formel I:

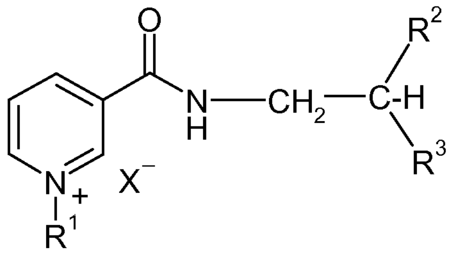

I

worin:

$R^1$ für $C_1$-$C_4$ alkyl steht;
$R^2$ für Wasserstoff, $C_1$-$C_4$ alkyl, $CH_2OH$ oder $CH_2ONO_2$ steht;
$R^3$ für $ONO_2$, $CH_2ONO_2$ oder OH steht;
vorausgesetzt, dass wenn $R^3$ für OH steht, dann $R^2$ für $CH_2ONO_2$ steht; und
$X^-$ ein organisches oder anorganisches Anion ist.

2. Verbindung nach Anspruch 1, worin $R^1$ für methyl steht.

3. Verbindung nach Anspruch 1, worin $R^1$ für methyl, $R^2$ für Wasserstoff, und $R^3$ für $ONO_2$, stehen.

4. Verbindung nach einem der Ansprüche 1 bis 3, worin X Cl ist.

5. Ein N'-Nitroxyalkylnikotinamid-Derivat nach den Ansprüchen 1 bis 4 zur Verwendung als Medikament.

6. Pharmazeutische Zusammensetzung, umfassend als Wirkstoff ein N'-Nitroxy-alkylnikotinamid-Derivat nach den Ansprüchen 1 bis 4, in Kombination mit einem pharmazeutisch verträglichen Träger und/oder Trägerstoffen.

7. Anwendung von eines N'-Nitroxyalkylnikotinamid-Derivates nach den Ansprüchen 1 bis 4 zur Herstellung eines Medikaments zur Behandlung von Erkrankungen des kardiovaskulären Systems.

8. Ein N'-Nitroxyalkylnikotinamid-Derivat nach den Ansprüchen 1 bis 4 zur Anwendung in der Behandlung oder Prophylaxe von Erkrankungen des kardiovaskulären Systems.

**9.** Anwendung nach Anspruch 7 oder 8, wobei die Erkrankung aus der Gruppe bestehend aus arterielle Hypertonie, koronare Herzkrankheit, akute und chronische ischämische Herzkrankheit, Arrhythmie, stabil und akuten Angina pectoris, Thrombose und periphere, zentrale, zerebrale und renale Durchblutungs Störungen ausgewählt ist.

## Revendications

**1.** Dérivé du N'-nitroxyalkylnicotinamide représentée par la formule generale I:

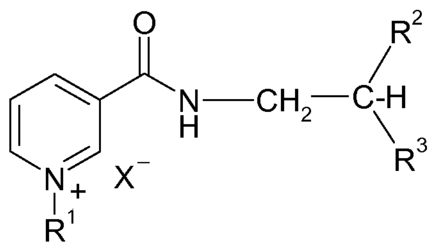

I

dans laquelle:

$R^1$ est alkyle en $C_1$-$C_4$;
$R^2$ est hydrogène, alkyle en $C_1$-$C_4$, $CH_2OH$ ou $CH_2ONO_2$;
$R^3$ est $ONO_2$, $CH_2ONO_2$ ou OH;
à condition que lorsque $R^3$ est OH, alors $R^2$ est $CH_2ONO_2$; et
$X^-$ est un anion organique ou inorganique.

**2.** Composé selon la revendication 1, dans lequel $R^1$ est méthyle.

**3.** Composé selon la revendication 1, dans lequel $R^1$ est méthyle, $R^2$ est hydrogène et $R^3$ est $ONO_2$.

**4.** Composé selon l'une quelconque des revendications 1 à 3 dans lequel X est Cl.

**5.** Dérivé du N'-nitroxyalkylnicotinamide telle que définie dans l'une quelconque des revendications 1 à 4 pour utilisation comme un médicament.

**6.** Composition pharmaceutique, comprenant comme une substance active une dérivé du N'-nitroxyalkylnicotinamide telle que définie dans l'une quelconque des revendications 1 à 4, en combinaison avec un support et/ou excipients pharmaceutiquement acceptables.

**7.** Utilisation d'une dérivé du N'-nitroxyalkylnicotinamide telle que définie dans l'une quelconque des revendications 1 à 4, dans la production d'un médicament pour le traitement et/ou prévention des maladies de système cardio-vasculaire.

**8.** Une dérivé du N'-nitroxyalkylnicotinamide telle que définie dans l'une quelconque des revendications 1 à 4, pour utilisation dans le procédé de traitement et/ou prévention des maladies de système cardio-vasculaire.

**9.** Utilisation selon revendication 7 ou 8 dans laquelle la maladie est choisie dans le groupe consistant en l'hypertension artérielle; la maladie cardiaque coronarienne; maladie cardiaque ischémique aiguë et chronique; l'arythmie; l'angine aiguë et chronique; la thrombose; et les disordres de flux sanguine périphérique, centrale, cérébrale et rénale.

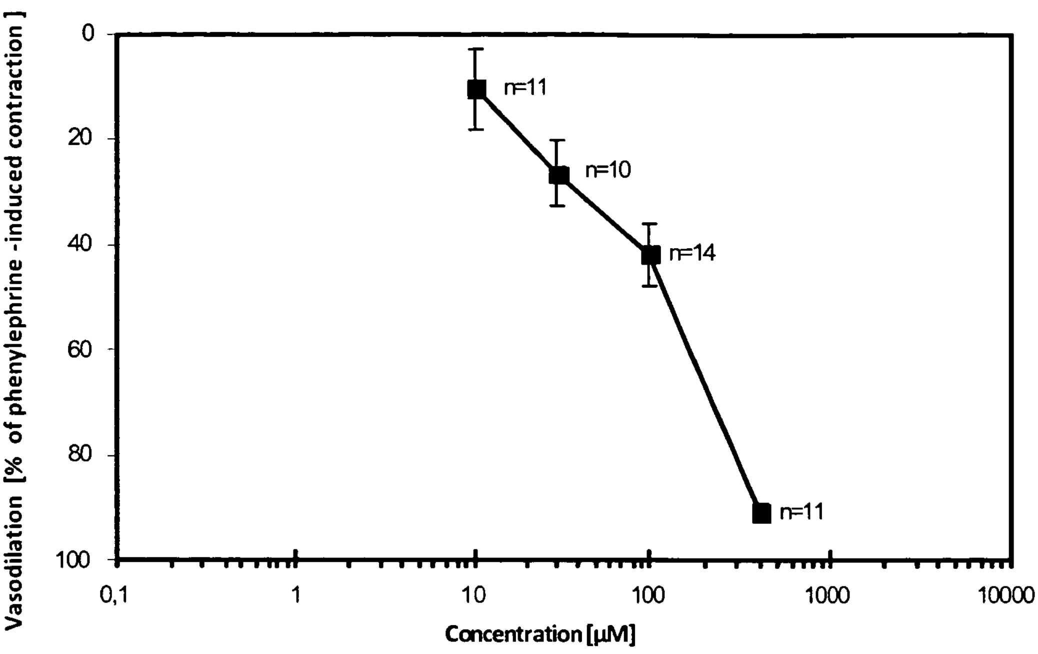


Fig. 1. The vasodilating effect of $MNIC^+Cl^-$ in isolated rat aorta

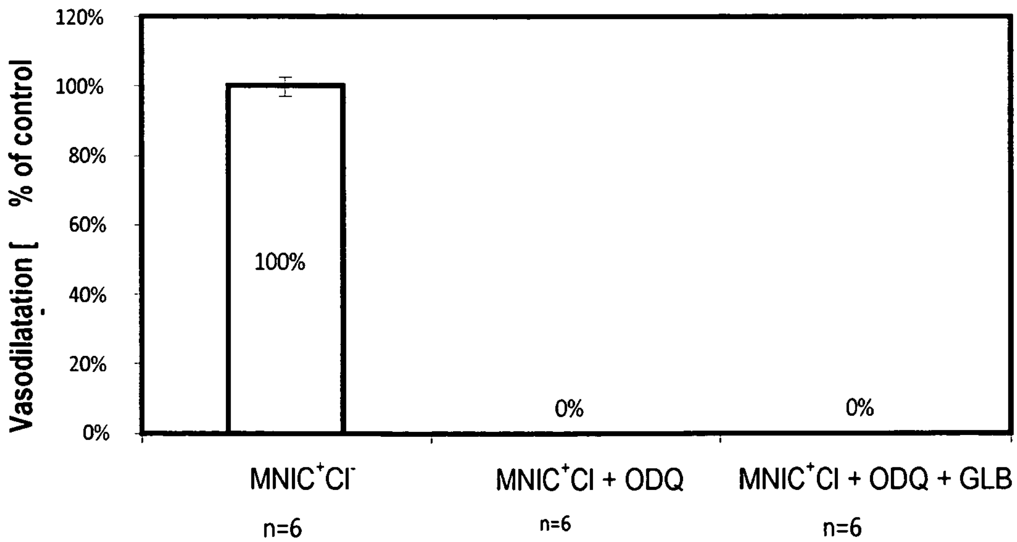


Fig. 2. Effect of ODQ on the vasodilating effect of $MNIC^+Cl^-$

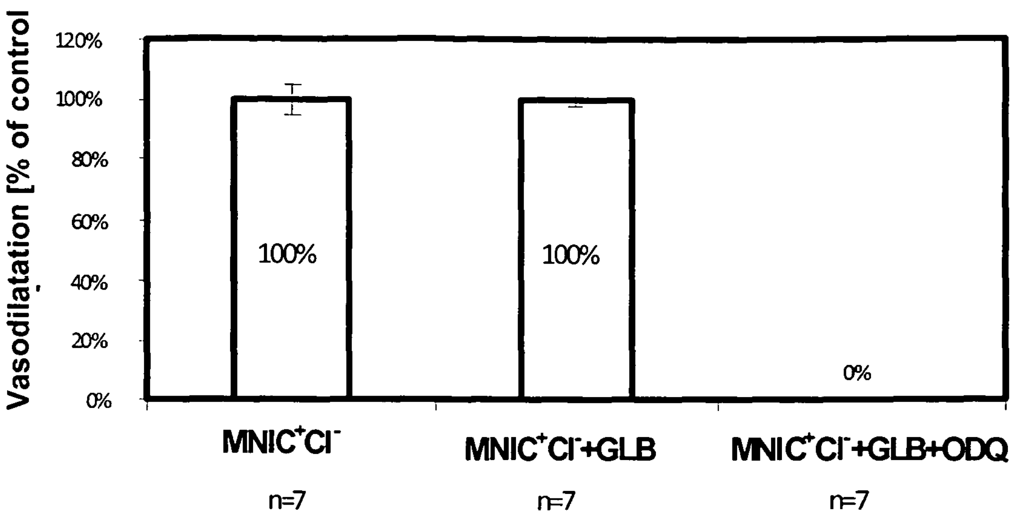


Fig. 3. Effect of GLB on the vasodilating effect of $MNIC^{+}Cl^{-}$

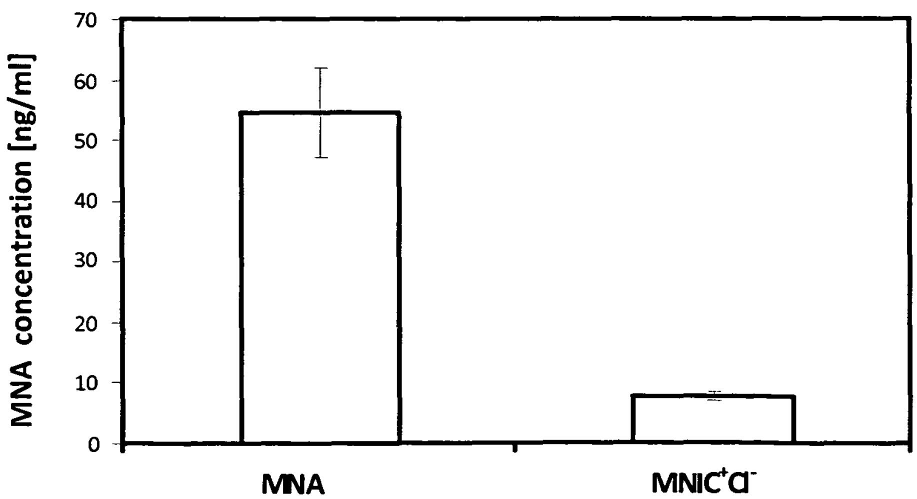


Fig. 4. Concentration of MNA in plasma after administration of MNA (left bar) or $MNIC^{+}Cl^{-}$ (right bar)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- DE 2714713
- WO 2000040559 A
- WO 2005067927 A
- AT 131118
- GB 348345 A
- US 3614408 A
- US 4115390 A


### Non-patent literature cited in the description


- **BUXTON, G.V. ; GREENSTOCK, C.L. ; HELMAN, W.P.** *J. Phys. Chem. Ref. Data,* 1988, vol. 17, 513
- **GEBICKI, J. ; SYSA-JEDRZEJOWSKA A. ; ADAMUS, J. ; WOŹNIACKA, A. ; RYBAK, M. ; ZIELONKA, J.** *Pol. J. Pharmacol.,* 2003, vol. 55, 109-112
- Remington's: The Science and Practice of Pharmacy. 2005